# EUROPÄISCHE PATENTANMELDUNG

(11) **EP 2 395 445 A2**
(43) Veröffentlichungstag der Anmeldung: **14.12.2011**
(21) Anmeldenummer: 11166849.7
(22) Anmeldetag: 20.05.2011
(51) Int. Cl.: G06F 19/00

(54) **Verfahren und Anordnung zur Erstellung eines individualisierten computergestützten Modells eines Systems sowie ein entsprechendes Computerprogramm und ein entsprechendes computerlesbares Speichermedium**

(30) Priorität: 09.06.2010 US 352836 P
(71) Anmelder: BIOTRONIK SE & Co. KG, 12359 Berlin (DE)
(72) Erfinder: Kirchner, Jens, 91052, Erlangen (DE); Urbaszek, Albrecht, 91336, Heroldsbach (DE)
(74) Vertreter: Lindner-Vogt, Karin L.

(57) **Zusammenfassung**

Die Erfindung betrifft ein Verfahren und eine Anordnung zur Erstellung eines individualisierten computergestützten Modells eines Systems sowie ein entsprechendes Computerprogramm und ein entsprechendes computerlesbares Speichermedium, welche insbesondere einsetzbar sind für eine Bestimmung physiologischer Variablen und/oder Parameter aus klinischen sowie kontinuierlichen Messungen. Des Weiteren wird durch die Erfindung eine Erkennung krankheitsbedingter Veränderungen insbesondere des Herzens und eine verbesserte medizinische Interpretation von Messungen von Implantatssensoren ermöglicht. Dabei ist die Erfindung nicht auf physiologische Systeme eingeschränkt, sondern kann vielmehr auch für eine Überwachung von technischen Systemen eingesetzt werden.

Hierfür wird vorgeschlagen, ein individualisiertes computergestütztes Modell eines Systems zu erstellen. Dabei wird ein initiales computergestütztes Modell des Systems erstellt, nachfolgend werden fortlaufend erfasste Messdaten ausgewertet und das individualisierte computergestützte Modell wird durch Modifizierung des initialen Modells in Abhängigkeit der erfassten Messdaten erstellt und angepasst.

## Beschreibung

Die Erfindung betrifft ein Verfahren und eine Anordnung zur Erstellung eines individualisierten computergestützten Modells eines Systems sowie ein entsprechendes Computerprogramm und ein entsprechendes computerlesbares Speichermedium, welche insbesondere einsetzbar sind für eine Bestimmung physiologischer Variablen und/oder Parameter aus klinischen sowie kontinuierlichen Messungen. Des Weiteren wird durch die Erfindung eine Erkennung krankheitsbedingter Veränderungen insbesondere des Herzens und eine verbesserte medizinische Interpretation von Messungen von Implantatssensoren ermöglicht. Dabei ist die Erfindung nicht auf physiologische Systeme eingeschränkt, sondern kann vielmehr auch für eine Überwachung von technischen Systemen eingesetzt werden.

### Hintergrund der Erfindung

Auf dem Gebiet der Erfindung sind bereits verschiedene Lösungen zur Auswertung kontinuierlich erfasster Daten vorgeschlagen worden, wie zum Beispiel die Pulskonturanalyse mit PiCCO-Monitor (Pulsion Medical Systems), die kontinuierliche Bestimmung des Cardiac Output mit dem Vigilance Monitor (Edwards Lifesciences) und eine Trendanalyse verschiedener aus IEGM und Impedanz abgeleiteter Parameter im HF-Prädiktor (Home Monitoring-Funktion).

Des Weiteren ist eine Simulation der Herzkontraktion mit Hilfe von Finite-Elemente-Modellen, z. B. im Karlsruhe Heart Model (MRI-Daten), oder eine Simulation der Blutzirkulation durch Kombination mehrerer Windkesselmodelle bekannt. Zellmodelle der Muskelkontraktion wurden ebenfalls schon vorgeschlagen.

Die bisherigen Methoden zur Analyse von Daten, die von Implantatsensoren geliefert werden, wie z. B. Impedanz oder Blutdruck, tragen individuellen Unterschieden zwischen den Patienten nicht oder nur bedingt Rechnung. So werden bei der Berechnung von Kenngrößen für benötigte Modellparameter wie auch für Schwellwerte, ab denen eine bestimmte Kenngröße auf krankhafte Veränderungen im Herzen hindeutet, Absolutwerte verwendet, die für alle Personen gleich sind. Teils können die interpersonellen Unterschiede beseitigt werden, indem relative Veränderungen bzgl. eines als individuell typisch angenommenen Wertes betrachtet werden. Patientenspezifische Informationen, die die Messungen wesentlich beeinflussen, v. a. Herzgeometrie, Lage der Sensoren (z. B. Elektroden), Dehnbarkeit der Arterien etc., bleiben dagegen unberücksichtigt.

Als Beispiel ist hier die Pulskonturanalyse zu nennen. Aufgabe dabei ist die Bestimmung des Schlagvolumens allein aus dem arteriellen Blutdrucksignal. Einfache Verfahren hierzu existieren, genauere Methoden aber verlangen die Kenntnis weiterer physiologischer Kenngrößen, z. B. des Dehnungsverhaltens der Arterie. Ein herkömmlicher Ansatz zur Beseitigung dieses Problems besteht in der Verwendung von Werten, die durch Mittelung über ein Patientenkollektiv bestimmt wurden. Allerdings variieren die in der Literatur angegebenen Werte z. B für die Compliance der Pulmonalarterie. zwischen einzelnen Patienten um mehr als einen Faktor 10, was die Vorhersagekraft für einen einzelnen Patienten deutlich beeinträchtigt. Ein anderer Ansatz besteht darin, die Größen durch Algorithmen auf der Basis von Näherungen oder Zusatzannahmen aus den vorhandenen Messsignalen zu errechnen. Ein Vergleich von Rekonstruktionsmethoden liefert beispielsweise Werte der pulmonalarteriellen Compliance, die sich um einen Faktor 3 unterscheiden. Es wird deutlich, dass die herkömmlichen Lösungen fehlerbehaftet bzw. -anfällig sind.

Auf der anderen Seite existieren Verfahren, z. B. aus der Bildgebung, die zwar sehr viele Informationen zur Verfügung stellen und damit eine präzise Abbildung der Herzkontraktion ermöglichen, die aber aufgrund des großen Messaufwandes nur einmalig oder in großen zeitlichen Abständen durchgeführt werden. Eine Anpassung an die sich verändernde Physiologie über längere Zeiträume hinweg und damit eine Überwachung des Patienten können sie daher nicht bieten.

So wurden speziell patientenspezifische Simulationen, insbesondere der Herzaktion (mit Hilfe von Finite-Elemente-Modellen) und/oder des Strömungsverhaltens des Blutes in den Herzkammern oder den Blutgefäßen vorgeschlagen. Als einer der umfassendsten Ansätze ist hier das Karlsruhe Heart Model zu nennen. Derartige Modelle beziehen ihr Datenmaterial typischerweise aus bildgebenden Verfahren und versprechen beispielsweise, bei atrialem Flimmern den Erfolg einer Ablation für unterschiedliche Loci abschätzen zu können. Da die Datenerhebung sehr aufwändig ist, sind diese Modelle darauf beschränkt, den aktuellen Zustand des Herzens darzustellen.

Die Aufgabe der vorliegenden Erfindung besteht somit darin, ein Verfahren und eine Anordnung zur Erstellung eines individualisierten computergestützten Modells eines Systems sowie ein entsprechendes Computerprogramm und ein entsprechendes computerlesbares Speichermedium bereitzustellen, welche die Nachteile der bekannten Lösungen vermeiden und insbesondere eine verbesserte Diagnose erlauben.

### Zusammenfassung der Erfindung

Diese Aufgabe wird erfindungsgemäß durch die Merkmale in den Ansprüchen 1 und 11 bis 15 gelöst. Zweckmäßige Ausgestaltungen der Erfindung sind in den Unteransprüchen enthalten.

Ein besonderer Vorteil des erfindungsgemäßen Verfahrens liegt darin, dass vorzugsweise patientenspezifische Parameter, die sich aus individuellen, ggf. pathologisch veränderten anatomischen und funktionellen Verhältnissen ergeben, individuell bestimmt werden und in ein Modell eingehen, welches zur Berechnung einer therapierelevanten physiologischen Größe, wie z. B. des Cardiac Output, aus einem Messsignal, wie z. B. dem pulmonalarteriellen Blutdruck, dient. Ohne die individuellen Parameter wäre nur eine grobe und relativ ungenaue Abschätzung möglich. Die Bestimmung der individuellen Parameter erfolgt vorzugsweise durch geeignete, klinisch praktikable Kalibrationsverfahren. Erst die Kenntnis dieser patientenspezifischen Parameter ermöglicht die Realisierung von Algorithmen zur Bestimmung physiologischer Kenngrößen, die an die individuellen Gegebenheiten des Patienten angepasst sind.

Es ist daher vorgesehen, dass bei dem erfindungsgemäßen Verfahren zur Erstellung eines individualisierten computergestützten Modells eines Systems ein initiales computergestütztes Modell des Systems erstellt und/oder angepasst wird. Vorzugsweise werden für die Erstellung des initialen Modells Daten genutzt, die aus einer umfangreichen, detaillierten Vermessung des Systems gewonnen werden. Da solche detaillierten Vermessungen in der Regel sehr aufwändig sind, wird erfindungsgemäß die Datenerfassung für eine detaillierte Vermessung nur einmal oder in größeren Zeitabschnitten, vorzugsweise im Abstand mehrerer Monate oder Jahre, durchgeführt. Bei der detaillierten Vermessung kann es sich beispielsweise um bildgebende Verfahren wie zum Beispiel Magnetic Resonance Imaging-(MRI-) oder Computertomographie- (CT-) Messungen handeln. Die Daten, mit denen das initiale Modell erstellt wird, können beispielsweise bei der Implantation eines Gerätes für die fortlaufende und/oder zeitweise fortlaufende Erfassung der Parameter erfasst werden. Dabei bezieht sich die Formulierung fortlaufende und/oder zeitweise fortlaufende Erfassung sowohl auf kontinuierliche Messungen, als auch auf Messungen, die in voreinstellbaren und/oder einstellbaren Abständen für eine voreinstellbare und/oder einstellbare Zeit vorgenommen werden. Vorzugsweise erfolgt die Speicherung, Analyse und Anpassung des Modells an zentraler Stelle, an der auch die Daten der Sensorsysteme des Implantats einlaufen. Alternativ oder parallel kann zumindest ein Teil der Speicherung, Analyse und Anpassung auch durch das Implantat übernommen werden.

Nach Erstellung des initialen Modells werden weiterhin fortlaufend, d.h. kontinuierlich oder in kurzen Zeitabständen, Messgrößen oder allgemein Parameter des Systems erfasst. In einer bevorzugten Realisierung werden die Signale täglich entweder über die kompletten 24 h oder einen geeigneten kürzeren Zeitraum, z.B. 30 min aufgezeichnet. Die fortlaufend erfassten Größen oder allgemein Parameter werden ausgewertet und vorzugsweise mit Referenzwerten verglichen. Eine bevorzugte Ausführungsform sieht vor, dass aus den erfassten Signalen oder allgemein Parametern Kenngrößen, wie z. B. das Schlagvolumen, die Wahrscheinlichkeit von Gewebe mit reduzierter Kontraktilität oder Orte von nekrosem Gewebe, bestimmt und diese Kenngrößen mit Referenzwerten verglichen werden. Das initiale Modell wird in Abhängigkeit des Ergebnisses des Vergleichs angepasst und so das individualisierte computergestützte Modell des Systems erstellt, oder es wird ein bereits individualisiertes computergestütztes Modell angepasst.

Eine bevorzugte Ausführungsform des erfindungsgemäßen Verfahrens sieht vor, dass es sich bei dem Modell um ein dynamisches Modell handelt. Hierfür kann beispielsweise ein geometrisches Modell mit einem Algorithmus verknüpft werden, welcher das (zeitliche) Systemverhalten beschreibt. Eine bevorzugte Ausführungsform sieht dabei vor, dass durch das Modell ein physiologisches System modelliert, dass also insbesondere anatomische und/oder funktionale Merkmale modelliert werden, und durch den Algorithmus physiologische Kenngrößen durch Simulation des realen Systems bestimmt werden, so dass die Simulation als Ausgangsgrößen physiologische Variablen und/oder Parameter bereitstellt. Im Falle physiologischer Modelle ist vorzugsweise vorgesehen, dass Sensoren zur fortlaufenden Erfassung der Messdaten als Implantatssensoren ausgeführt sind.

Eine mögliche Realisierung der vorliegenden Erfindung besteht darin, dass eine Modellierung der Herzaktion auf der Basis entweder einer einzelnen klinischen Messung oder mehrerer in großen zeitlichen Abständen durchgeführten Datenerfassungen erfolgt und eine kontinuierliche Anpassung des Modells mit Hilfe von Sensordaten eines Implantats vorgenommen wird. Das so angepasste Modell wird für die Bestimmung diagnostisch relevanter Parameter eingesetzt, die die Entstehung oder Verschlechterung kardialer Erkrankungen anzeigen.

Bei dem Modell kann es sich zum Beispiel um ein Modell zumindest von Teilen des Herzkreislaufsystems, wie z. B. der Myokardgeometrie, um ein Modell eines Gefäßsystems, insbesondere um ein Modell von Gefäßverzweigungen, um ein Modell der Viskosität und des Strömungsprofils des Blutes, um ein Modell der örtlichen Position von Sensoren zur fortlaufenden Erfassung der Messdaten o. dgl. handeln.

Mit dem Modell können beispielsweise Herzaktionen, wie Myokardkontraktionen, das Dehnungsverhalten von Gefäßen, das Strömungsverhalten von Flüssigkeiten (in den Gefä-βen), intrazelluläre Prozesse o. dgl. simuliert werden.

Eine bevorzugte Ausführungsform sieht vor, dass das Modell als Finite-Elemente-Modell realisiert wird.

In einer bevorzugten Ausführungsform ist vorgesehen, dass das initiale Modell angepasst, insbesondere optimiert wird, indem nachfolgend fortlaufend erfasste Messgrößen oder allgemein Parameter bzw. aus den Messgrößen oder allgemein Parametern ermittelte Kenngrößen mit Variablen oder allgemein Parametern bzw. Kenngrößen verglichen werden, die aus dem Modell z. B. mittels Simulation gewonnen wurden. In Abhängigkeit des Vergleichsergebnisses, das beispielsweise ein Ähnlichkeitswert sein kann, werden Parameter des Modells variiert und das Modell so an die aktuellen Verhältnisse angepasst. Bei den Messgrößen oder allgemein Parametern kann es sich beispielsweise um den Blutdruck oder die Impedanz und/oder bei der Kenngröße um das Schlagvolumen handeln. In einer bevorzugten Ausführungsform ist vorgesehen, dass freie Parameter an die gemessenen Größen oder allgemein Parameter bzw. an die aus den Signalen oder allgemein Parametern ermittelten Kenngrößen gefittet werden.

Als besonders vorteilhaft erweist es sich, dass das aktualisierte individualisierte Modell für die Diagnoseerstellung genutzt wird. Hierfür ist in einer bevorzugten Ausführungsform vorgesehen, dass die aktualisierten Parameter einem Klassifikator zugeführt werden. Eine Anordnung nach der Erfindung weist mindestens einen Chip und/oder Prozessor auf und ist derart eingerichtet, dass ein Verfahren zur Erstellung eines individualisierten computergestützten Modells eines Systems ausführbar ist, wobei ein initiales computergestütztes Modell des Systems erstellt wird, nachfolgend fortlaufend erfasste Messdaten ausgewertet werden und das individualisierte computergestützte Modell durch Modifizierung des initialen Modells in Abhängigkeit der erfassten Messdaten erstellt bzw. nachgeführt wird.

Ein Computerprogramm zur Modellerstellung ermöglicht es einer Datenverarbeitungseinrichtung, nachdem es in den Speicher der Datenverarbeitungseinrichtung geladen worden ist, ein Verfahren zur Erstellung eines individualisierten computergestützten Modells eines Systems durchzuführen, wobei ein initiales computergestütztes Modell des Systems erstellt wird, nachfolgend fortlaufend erfasste Messdaten ausgewertet werden und das individualisierte computergestützte Modell durch Modifizierung des initialen Modells in Abhängigkeit der erfassten Messdaten erstellt wird oder ein bereits individualisiertes computergestütztes Modell angepasst wird.

In einer weiteren bevorzugten Ausführungsform der Erfindung ist vorgesehen, dass das erfindungsgemäße Computerprogramm modular aufgebaut ist, wobei einzelne Module auf verschiedenen Datenverarbeitungseinrichtungen installiert sind.

Vorteilhafte Ausführungsformen sehen zusätzlich Computerprogramme vor, durch welche weitere in der Beschreibung angegebene Verfahrensschritte oder Verfahrensabläufe ausgeführt werden können.

Solche Computerprogramme können beispielsweise (gegen Gebühr oder unentgeltlich, frei zugänglich oder passwortgeschützt) downloadbar in einem Daten- oder Kommunikationsnetz bereitgestellt werden. Die so bereitgestellten Computerprogramme können dann durch ein Verfahren nutzbar gemacht werden, bei dem ein Computerprogramm nach Anspruch 13 aus einem elektronischen Datennetz, wie beispielsweise aus dem Internet, auf eine an das Datennetz angeschlossene Datenverarbeitungseinrichtung heruntergeladen wird.

Um das erfindungsgemäße Verfahren durchzuführen, ist vorgesehen, ein computerlesbares Speichermedium einzusetzen, auf dem ein Programm gespeichert ist, das es einer Datenverarbeitungseinrichtung ermöglicht, nachdem es in den Speicher der Datenverarbeitungseinrichtung geladen worden ist, ein Verfahren zur Erstellung eines individualisierten computergestützten Modells eines Systems durchzuführen, wobei ein initiales computergestütztes Modell des Systems erstellt wird, nachfolgend fortlaufend erfasste Messdaten ausgewertet werden und das individualisierte computergestützte Modell durch Modifizierung des initialen Modells in Abhängigkeit der erfassten Messdaten erstellt wird, oder ein bereits individualisiertes computergestütztes Modell angepasst wird.

Die Erfindung stellt ein Computermodell zur Bestimmung physiologischer Variablen und Parameter aus klinischen sowie kontinuierlichen Messungen bereit. Dabei werden die Vorteile von zwei Methoden zur Diagnose und Vorhersage kardialer Erkrankungen miteinander kombiniert: eine einmalige detailreiche Erfassung der Herzgeometrie und des Kontraktionsverhaltens, z. B. aus MRI- oder CT-Messungen, mit einer kontinuierlichen Aufzeichnung einfacher Messgrößen wie Impedanz und Blutdruck zur ständigen Überwachung des Patienten. Durch letztgenannte Daten wird ein einmalig, mit einer aufwändigen Datenerfassung erstelltes Modell der Herzkontraktion über die Zeit hinweg an eine sich verändernde Physiologie angepasst. Gleichzeitig erlaubt ein solches erfindungsgemäßes Modell eine detaillierte Interpretation der Sensordaten auf einer patientenspezifischen Informationsbasis, was eine Verbesserung der bisher verwendeten und die Entwicklung neuer Prädiktoren erlaubt.

Durch die Erfindung wird insbesondere die Erkennung krankheitsbedingter Veränderungen des Herzens und einer damit einhergehenden Verschlechterung von dessen Leistungsfähigkeit verbessert. Die Erfindung kann weiter vorteilhaft eingesetzt werden zur Prädiktion von Arrhythmien. Darüber hinaus wird eine Verbesserung der medizinischen Interpretation von Messungen der Implantatsensoren auf einer patientenspezifischen Informationsbasis erreicht, was insbesondere zu einer genaueren Diagnose führt und die Planung medizinischer Eingriffe unterstützen kann. Durch die Erfindung werden mehr Informationen über den Patienten bereitgestellt, sodass Prädiktoren spezifischer und damit genauer arbeiten können. Ein erfindungsgemäß erstelltes Modell stellt ein zusätzliches Feature im Home Monitoring für den behandelnden Arzt dar und liefert unterstützende Informationen für eine Therapieentscheidung. So kann ein Warnsignal an den Arzt übermittelt werden, und/oder Anweisungen an Patienten über ein Patientengerät und/oder externes Gerät übermittelt werden, z. B. bezüglich einer Medikamenteneinnahme und/oder Kontaktaufnahme zum Arzt und/oder andere Verhaltensweisen. Auch ist die Darstellung der abgeleiteten Parameter und/oder der abgeleiteten Diagnose und/oder der abgeleiteten Therapievorschläge und/oder des Krankheits- und/oder Medikamentenmonitorings im Programm, HMSC und/oder einen externen Gerät möglich.

Figuren:
- Fig. 1: zeigt ein Flussdiagramm zur Veranschaulichung einer Ableitung von diagnostisch relevanten Kennzahlen;
- Fig. 2: zeigt ein Schema zur Anpassung der Modellparameter an Veränderungen im gemessenen Signal.

Die Erfindung wird nachfolgend anhand eines Ausführungsbeispiels näher erläutert.

Die Erfindung soll nachfolgend beispielhaft am Modell kardiologischer Prozesse näher erläutert werden. Ein beispielhafter Algorithmus zur Berechnung physiologischer Größen speist sich aus zwei Datenquellen: permanent einlaufenden Sensordaten (z. B. im Rahmen von Home Monitoring) und einer ausführlichen, einmalig (z. B. bei der Implantation) oder in großen Abständen während Follow-ups durchgeführten Datenerfassung. Die so bestimmten Kenngrößen erlauben dann ein Patientenmonitoring mit hoher Verlässlichkeit.

In einer beispielhaften Ausführungsform der Erfindung wird ein patientenspezifisches Modell mithilfe einer einmaligen klinischen Messung (oder mehreren in längeren zeitlichen Abständen) aufgestellt und mit Messdaten vom Implantatsensorsystem über die Zeit hinweg angepasst. Ein solches System ist mit unterschiedlichen Graden an Komplexität und mit unterschiedlichen Zielsetzungen realisierbar: In den Algorithmus zur Bestimmung des Schlagvolumens des Herzens können zusätzlich zur Compliance auch andere Elemente implementiert werden, so die Viskosität und das Strömungsprofil des Blutes oder Gefäßverzweigungen, die zu Pulswellenreflexionen führen. Neben der Pulmonalarterie ließen sich weitere Teile des Gefäßsystems simulieren, wie verschiedentlich, wenngleich noch nicht adaptiv und patientenspezifisch, in Mehrkompartimentenmodellen geschehen. In der gleichen Weise, wie sich die Teile des Herzkreislaufsystems variieren lassen, die durch ein derartiges Modell erfasst werden, kann ein solches auch unterschiedliche Skalenbereiche abdecken und bis zu intrazellulären Prozessen reichen.

Ein derartiges individuelles, adaptives System vereinigt die Vorteile einer einmaligen, umfangreichen Messung mit denen einer kontinuierlichen Messung einer einfachen Messgröße. Methoden, die bislang nur auf die Informationen eines einzelnen Messsignals beschränkt waren, können nun auf einen weitaus größeren und v. a. individuellen Datenpool zurückgreifen, was eine deutliche Verbesserung ihrer Genauigkeit und damit ihrer Detektions- und Prädiktionsmöglichkeit bedeutet. Änderungen in Form, Amplitude und Offset der Sensordaten können besser bestimmten physiologischen Mechanismen zugewiesen und so evtl. eine krankhaft veränderte Herzgeometrie frühzeitig detektiert werden. Schließlich wären Simulationen des Systemverhaltens nach einem medizinischen Eingriff durchführbar, wodurch sich Risiken und Heilungschancen abschätzen ließen.

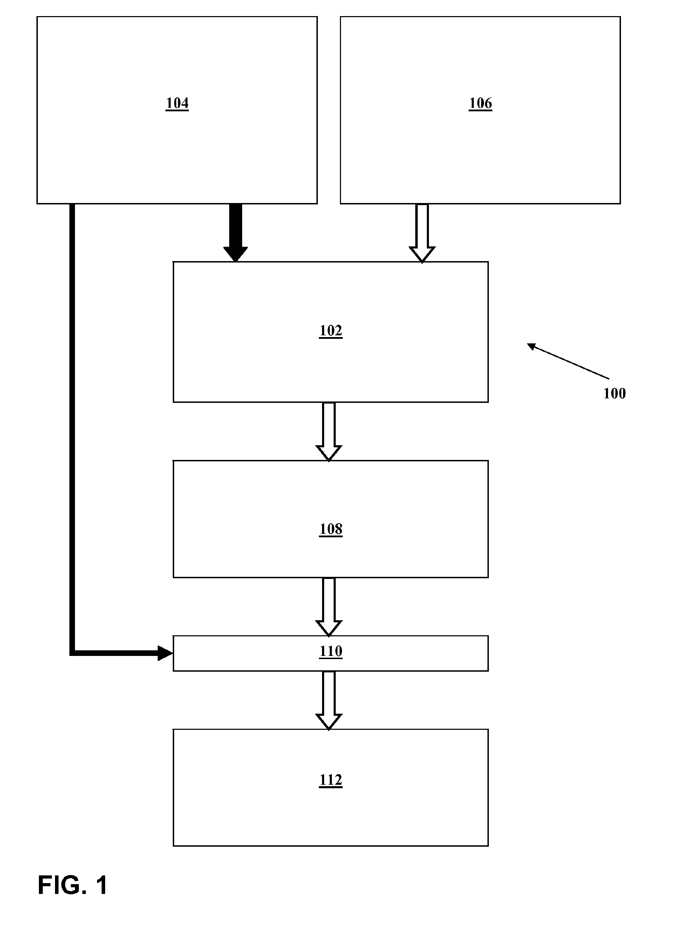

Anhand von Figur 1 soll der Prozess 100 der Ableitung von diagnostischen Kennzahlen beispielhaft erläutert werden. Schwarze, gefüllte Pfeile bezeichnen dabei einen einmaligen (oder in großen zeitlichen Abständen erfolgenden) Datenfluss, weiße Pfeile mit schwarzem Rand hingegen kontinuierlich bzw. in kurzen regelmäßigen Abständen ablaufende Prozesse. Basierend auf einer umfangreichen Datenmenge, die ein Herzkreislaufsystem detailliert beschreiben, wird ein initiales Modell des Herzkreislaufsystems erstellt (Schritt 102). Eine bevorzugte Ausführungsform sieht vor, dass ein zeitadaptives komplexes Modell des Herzkreislaufsystems erstellt wird. Ein solches zeitadaptives komplexes Modell kann beispielsweise eine Pulskonturanalyse, eine Simulation der Erregungsausbreitung, eine Blutflusssimulation und/oder eine Kontraktionssimulation umfassen.

Dieses initiale Modell des Herzkreislaufsystems beruht auf der einmaligen Erfassung 104 von Daten, welche das System detailliert beschreiben. Bei diesen Daten kann es sich beispielsweise um
d ie Geometrie des Myokards,
d ie Faserrichtung des Myokards,
d ie Erregungsausbreitung auf dem Myokard,
d ie Lage von Elektroden eines Implantats,
d ie Geometrie des arteriellen Gefäßsystems und/oder
d ie Compliance der arteriellen Gefäße handeln.

Die einmalige Erfassung 104 der Daten erfolgt beispielsweise mithilfe von bildgebenden Verfahren wie MRI- oder CT-Messungen.

Für die Anpassung des initialen Modells des Herzkreislaufsystems erfolgt eine kontinuierliche Messung 106 von Größen oder allgemein Parametern des Herzkreislaufsystems, wie z. B. Impedanz, Blutdruck und/oder die Aufnahme eines intrakardialen Elektrogramms (IEGM, Intra Cardiac Electrogram).

Aus den Daten, die durch die kontinuierliche Messung 106 gewonnen werden, werden Kennzahlen abgeleitet (Schritt 108). So kann beispielsweise als Kennzahl das Schlagvolumen aus dem arteriellen Blutdruck bestimmt werden. Als weitere Kennzahlen können beispielsweise die Wahrscheinlichkeit von Gewebe mit reduzierter Kontraktilität oder Orte von nekrosem Gewebe betrachtet werden (Einzelheiten hierzu werden weiter unten näher erläutert).

Die Kennzahl(en) wird (werden) in Schritt 110 mit Referenzwerten verglichen. Diese Referenzwerte können komplett oder zum Teil während der initialen Messung 104, z. B. durch Messungen unter definierten physiologischen Bedingungen (beispielsweise in Ruhe/unter Belastung, mit intrinsischem/stimuliertem Rhythmus oder unter Medikamentengabe), bestimmt worden sein. In Abhängigkeit des Ergebnisses des Vergleichs werden in Schritt 112 Systemzustände signalisiert. Dies kann beispielsweise durch Anzeige in einem Fernüberwachungssystem wie beispielsweise dem Home Monitoring Service Center (HMSC), durch Anzeige in einem externen medizinischen Gerät oder dgl. erfolgen. Alternativ oder zusätzlich können auch in Abhängigkeit des Ergebnisses des Vergleichs Implantatseinstellungen (automatisch) geändert oder Empfehlungen an einen Arzt gegeben werden.

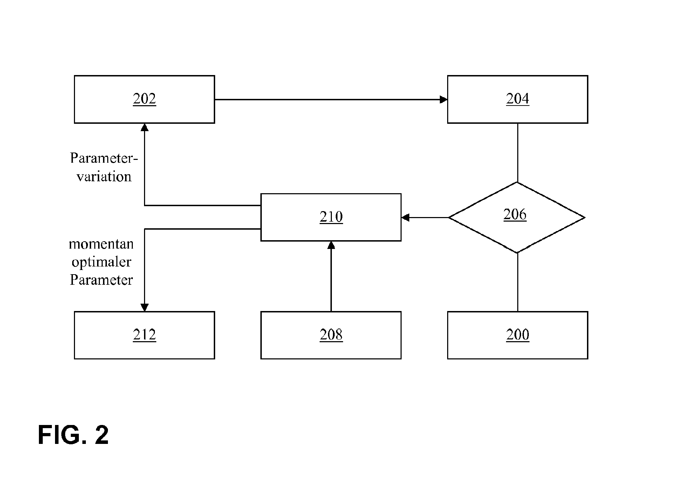

In Figur 2 wird die Anpassung von Modellparametern an Veränderungen in gemessenen Signalen veranschaulicht. Für die Anpassung, insbesondere eine Optimierung, der Parameter werden die (kontinuierlich) gemessenen Signale 200 mit den entsprechenden, mithilfe des Modells 202 simulierten Signalen 204 verglichen und ein Maß 206 für Übereinstimmung von gemessenem 200 und durch Simulation gewonnenem Signal 204 ermittelt. Bei dem Modell 202 kann es sich beispielsweise um ein Modell der Kontraktion des Myokards und des Blutflusses handeln. Als gemessene 200 bzw. simulierte Signale 204 könnten in diesem Falle beispielsweise der Blutdruck und die intrakardiale Impedanz ausgewertet werden; das Maß 206 für die Übereinstimmung kann beispielsweise ermittelt werden, indem die Kurvendifferenz über einen Zyklus integriert wird. Für die Parametervariation werden bestimmte Vorgaben 208 für Parameter bereitgehalten, die variiert werden können, wie beispielsweise im Falle des Modells 202 des Kontraktionsverhaltens Loci von potentiell unterversorgtem Gewebe. In Abhängigkeit des Maßes 206 an Übereinstimmung und der Vorgaben 208 für die Parametervariation erfolgt eine Optimierung 210 der Parameter des Modells 202. Die aktuellen optimalen Parameter werden einer Auswerteeinheit, beispielsweise einem Klassifikator 212, zur Diagnoseerstellung zugeführt. In dem speziellen Fall des Modells 202 des Kontraktionsverhaltens könnte beispielsweise ein Befund darüber erstellt werden, ob ein geringes, mittleres oder hohes Herzinsuffizienzrisiko vorliegt.

Durch den regelmäßigen oder sogar kontinuierlichen Abgleich mit Sensordaten 200 wie Impedanz oder Blutdruck, die von einem Implantat aufgezeichnet und im Rahmen des Home Monitorings zur weiteren Auswertung übertragen werden, wird das Modell 202 nachgeführt. Durch die Optimierung der Simulation auf die Messdaten hin kann eine Veränderung in der Herzgeometrie bzw. der Erregungsleitung festgestellt, die weitere Entwicklung interpoliert und mögliche Komplikationen frühzeitig vorhergesagt werden.

Auch ist ein Medikamentenmonitoring möglich. Bei Diuresepatienten wird sich ein erhöhtes/vermindertes Blutvolumen insbesondere im Blutdruck zeigen. Ferner ließen sich Medikamente, die in den Ionenhaushalt der Zelle eingreifen, über ein Zellmodell in das System einkoppeln.

Dabei sind je nach verwendetem Modell 202 unterschiedliche Formen der Parameteroptimierung möglich, u. a.:
Parameterschätzverfahren
Trial-and-Error-Verfahren

Hier wird getestet, ob eine Veränderung im Signalverlauf sich durch ein oder mehrere Elemente eines vordefinierten Satzes potentieller Erkrankungen "erklären" lässt. Bei einem Modell 202, das das Kontraktionsverhalten und den Blutfluss simuliert, können z. B. mehrere Myokardareale vordefiniert werden, deren Kontraktilität bei verminderter Blutversorgung sinkt. Bei der Parameteroptimierung 210 wird dann getestet, ob eine Reduktion der Kontraktilität in Stufen von z. B. 25% in einem der Areale oder einer Kombination davon die gemessenen Blutdruck- und intrakardialen Impedanzsignale 200 simulieren kann.

Aufgrund der Komplexität des Modells 202 erfolgt die Datenverarbeitung vorzugsweise nicht im Implantat, das die kontinuierlichen Daten 200 liefert, sondern in einem externen Gerät. Hierfür sind zwei Möglichkeiten parallel oder alternativ vorgesehen:
1. Servicezentrum
   Übertragung der Daten 200 an ein externes Zentrum zur Weiterverarbeitung
2. Externes Gerät
   z. B. stationäre Patientenüberwachung; Unterstützung der Implantatprogrammierung.

Abhängig von der Realisierung des Systems zur Datenverarbeitung sind als Schnittstelle zum Arzt oder zum Patienten folgende Möglichkeiten parallel oder alternativ vorgesehen:
1.1 Anzeige von Kennzahlen oder deren Trends im HMSC,
1.2 Ausgabe von Warnsignalen, falls ein Schwellwert überschritten wird (im HMSC, per SMS an den behandelnden Arzt),
2.1 Anzeige von Kennzahlen in einem externen Gerät,
2.2 Vorschlag für Parametereinstellungen eines Implantats in einem externen Gerät.
Nachfolgend soll die oben skizzierte Funktionsweise der Erfindung in größerem Detail beschrieben werden:

### Berechnung des Schlagvolumens

Anstelle von Methoden, die zur Berechnung des Schlagvolumens (SV) ausschließlich den arteriellen Blutdruck verwenden, ließen sich bei einer einmaligen Messung 104, die beispielsweise bei der Implantation des Drucksensors erfolgt, wichtige Größen des betroffenen Gefäßsystems messen wie beispielsweise das Impedanzspektrum oder die Compliance. Mit dem hierdurch realisierbaren Modell 202 zur SV-Bestimmung ließen sich z. B. oszillatorische Komponenten des Blutflusses erfassen.

Weitere Diagnosemöglichkeiten eröffnen sich durch Kombination eines Gefäßmodells mit einer Blutdruckmessung: Durch Detektion reflektierter Druckwellen im Signal und Kenntnis der Reflexionsorte respektive der zurückgelegten Wegstrecken aus dem Gefäßmodell ließe sich die Pulswellengeschwindigkeit abschätzen.

### Intrakardiale Impedanzmessungen

Neben der weiter unten beschriebenen Integration einer intrakardialen Impedanzmessung in ein Blutfluss- oder Kontraktionsmodell 202 des Herzens ließe sich durch Kenntnis der Lage der Elektroden eine bessere Interpretation des Signals 200 erreichen. So ließe der Impedanzwert nicht nur auf Änderungen relativ zum stromführenden Volumenobjekt schließen, sondern dieses ließe sich zusätzlich mit dem gesamten Kammervolumen in Verbindung und/oder Verhältnis setzen.

### Detektion von Gewebeveränderungen

Messungen von IEGM, intrakardialer Impedanz und Blutdruck stellen das Ergebnis von Erregungsleitung bzw. Kontraktion des Myokards dar und damit eine Art Projektion dieser komplexeren Signalentwicklung auf einfache Messgrößen. Ausgehend von einem Modell 202, das beispielsweise Myokardgeometrie und Erregungsleitung mit dem resultierenden IEMG verbindet, ließen sich langfristige Änderungen im IEGM auf Änderungen im erregungsleitenden Gewebe zurückführen. Entsprechendes gilt für Änderungen im Kontraktionsverhalten des Myokards, die durch Impedanz- oder/und Blutdruckmessung aufgedeckt werden könnten.

So wird entsprechend Figur 2 durch Variation der Parameter, die die Gefäß- oder Kontraktionseigenschaften beschreiben, eine Änderung im gemessenen Signal 200 auf eine Änderung im komplexen physiologischen Modell 202 zurückgeführt. Der Optimierungsalgorithmus bestimmt die neuen Parameter dabei darauf hin, dass simuliertes, d. h. aus dem Modell abgeleitetes, Signal 204 und gemessenes Signal 200 möglichst übereinstimmen. Gegenüber den bisherigen Methoden, z. B. zur Detektion von Kontraktilitätseinbußen allein aus den genannten Messgrößen, ergeben sich mehrere Vorteile:
Hohe Sensitivität und Spezifität bei den verwendeten Methoden, da Fehldetektionen und nicht bzw. zu spät erkannte Ereignisse aufgrund von Patientenbesonderheiten gerade durch die stärkere Ankopplung an die Physiologie vermieden werden.
Ein solches komplexeres Modell lässt sich um weitere Sensorgrößen erweitern.
Da Veränderungen in den kontinuierlich gemessenen Signalen 200 wieder auf die Physiologie zurückgeführt werden, lässt sich der Gesundheitszustand wie auch die Erfolgsaussichten spezieller Therapiemöglichkeiten abschätzen.

### Bezugszeichenliste:

- 100: Prozess der Ableitung von diagnostischen Kennzahlen
- 102: Erstellung des Modells
- 104: einmalige Erfassung von Daten
- 106: kontinuierliche Messung von Parametern
- 108: Ableitung von Kennzahlen
- 110: Vergleich mit Referenzwerten
- 112: Signalisierung von Systemzuständen
- 200: gemessenes Signal
- 202: Modell
- 204: simuliertes Signal
- 206: Maß der Übereinstimmung
- 208: Vorgaben für die Parametervariation
- 210: Parameteroptimierung
- 212: Klassifikator

## Patentansprüche

1. Verfahren zur Erstellung eines individualisierten computergestützten Modells (202) eines Systems, wobei
ein initiales computergestütztes Modell des Systems erstellt wird,
nachfolgend fortlaufend und/oder zeitweise fortlaufend erfasste Messdaten (200) ausgewertet werden und
das individualisierte computergestützte Modell (202) durch Modifizierung des initialen Modells in Abhängigkeit der erfassten Messdaten (200) erstellt und/oder angepasst wird.

2. Verfahren nach Anspruch 1, **dadurch gekennzeichnet, dass** das Modell (202) mit einem Algorithmus zur Simulation des Systemverhaltens verknüpft wird.

3. Verfahren nach Anspruch 1 oder 2, **dadurch gekennzeichnet, dass** mit dem Modell (202) ein physiologisches System modelliert wird.

4. Verfahren nach Anspruch 3, **dadurch gekennzeichnet, dass** mit dem Modell (202) ein Herzkreislaufsystem oder Gefäßsystem oder Teile davon modelliert wird.

5. Verfahren nach einem der voranstehenden Ansprüche, **dadurch gekennzeichnet, dass** nachfolgend erfassten Messdaten (200) von Implantatssensoren bereitgestellt werden.

6. Verfahren nach einem der voranstehenden Ansprüche, **dadurch gekennzeichnet, dass** das initiale Modell modifiziert wird, indem zumindest ein Teil der nachfolgend erfassten Messdaten (200) oder aus den nachfolgend erfassten Messdaten (200) gewonnene Daten mit aus der Simulation gewonnenen Werten (204) verglichen und in Abhängigkeit des Ergebnisses des Vergleichs Parameter des Modells (202) variiert werden.

7. Verfahren nach Anspruch 6, **dadurch gekennzeichnet, dass** das initiale Modell modifiziert wird durch Fitten freier Parameter an die nachfolgend erfassten Messdaten (200) oder an die aus den nachfolgend erfassten Messdaten (200) gewonnenen Daten.

8. Verfahren nach Anspruch 6 oder 7, **dadurch gekennzeichnet, dass** für die Modifizierung des initialen Modells Parameterschätzverfahren und/oder Trial-and-Error-Verfahren genutzt werden.

9. Verfahren nach einem der voranstehenden Ansprüche, **dadurch gekennzeichnet, dass** das initiale Modell durch Auswertung von Daten erstellt wird, die durch eine detaillierte Vermessung des Systems gewonnen werden.

10. Verfahren nach Anspruch 9, **dadurch gekennzeichnet, dass** die detaillierte Vermessung bildgebende Verfahren wie Röntgen, Sonographie, Szintigraphie, PET, Magnetic Resonance Imaging und/oder Computertomographie umfasst.

11. Verfahren zur Diagnoseerstellung, wobei ein Modell (202) eingesetzt wird, das mit einem Verfahren gemäß einem der Ansprüche 1 bis 10 erstellt wurde.

12. Anordnung mit mindestens einem Chip und/oder Prozessor, wobei die Anordnung derart eingerichtet ist, dass ein Verfahren zur Erstellung eines individualisierten computergestützten Modells (202) eines Systems gemäß einem der Ansprüche 1 bis 10 ausführbar ist.

13. Computerprogramm, das es einer Datenverarbeitungseinrichtung ermöglicht, nachdem es in Speichermittel der Datenverarbeitungseinrichtung geladen worden ist, ein Verfahren zur Erstellung eines individualisierten computergestützten Modells (202) eines Systems gemäß einem der Ansprüche 1 bis 10 durchzuführen.

14. Computerlesbares Speichermedium, auf dem ein Programm gespeichert ist, das es einer Datenverarbeitungseinrichtung ermöglicht, nachdem es in Speichermittel der Datenverarbeitungseinrichtung geladen worden ist, ein Verfahren zur Erstellung eines individualisierten computergestützten Modells (202) eines Systems gemäß einem der Ansprüche 1 bis 10 durchzuführen.

15. Verfahren, bei dem ein Computerprogramm nach Anspruch 13 aus einem elektronischen Datennetz, wie beispielsweise aus dem Internet, auf eine an das Datennetz angeschlossene Datenverarbeitungseinrichtung heruntergeladen wird.
